# EUROPEAN PATENT APPLICATION

(11) **EP 4 847 032 A1**
(43) Date of publication of application: **02.12.2026**
(21) Application number: 25745349.8
(22) Date of filing: 23.01.2025
(51) Int. Cl.: A61K 38/08, A61P 25/28, A23L 33/18

(54) **USE OF PEPTIDE AS THERAPEUTIC AGENT FOR DEGENERATIVE BRAIN DISEASES**

(30) Priority: 24.01.2024 KR 20240011114
(71) Applicant: Kine Sciences Co., Ltd., Seongnam-si Gyeonggi-do, 13494 (KR)
(72) Inventor: CHO, Daeho, Seongnam-si Gyeonggi-do, 13494 (KR); GU, Changkyu, Seoul 03325 (KR); RYU, In Hyun, Seoul 02585 (KR); LEE, Heeeun, Seoul 08302 (KR); KANG, Jun-il, Seongnam-si Gyeonggi-do 13494 (KR); CHA, Hye-Lim, Yongin-si Gyeonggi-do 16989 (KR)
(74) Representative: Vossius & Partner Patentanwälte Rechtsanwälte mbB
(86) International application number: PCT/KR2025/001311
(87) International publication number: WO 2025/159525

(57) **Abstract**

The present invention relates to the use of a peptide as a therapeutic agent for degenerative brain diseases. More specifically, it was confirmed that the peptide of the present invention targets microglia and induces inflammatory microglia to an anti-inflammatory state under disease conditions, thereby reducing neuroinflammation and increasing phagocytic activity to remove amyloid beta protein. In addition, in an animal model of a degenerative brain disease, the peptide was confirmed to exhibit effects of reducing amyloid beta, restoring synaptic plasticity, and improving cognitive impairments associated with anxiety, learning, and memory. Therefore, the peptide of the present invention may be usefully employed as an active ingredient in a composition for preventing or treating degenerative brain diseases and cognitive impairment, learning impairment, or memory impairment associated therewith.

## Description

### [Technical Field]

The present invention relates to the use of a peptide as a therapeutic agent for degenerative brain diseases, and more particularly, to the use of a trimeric peptide consisting of the amino acid sequence represented by SEQ ID NO: 1 as a therapeutic agent for degenerative brain diseases.

### [Background Art]

Degenerative brain diseases refer to disorders occurring in the brain among degenerative diseases associated with aging, and may be classified according to major symptoms and affected brain regions. Representative examples include Alzheimer's disease and Parkinson's disease. Degenerative brain diseases are known to be caused by neuronal cell death resulting from protein aggregation induced by neurodegeneration associated with aging and genetic and environmental factors.

In addition, degenerative brain diseases involve the death or degeneration of specific brain cells, which may progress either temporarily or over a prolonged period. Since dead brain cells cannot be regenerated, such degeneration ultimately leads to irreversible loss of brain function. In particular, brain dysfunction accompanied by progressive impairment of cognitive function, sensory function, motor function, and systemic function ultimately results in changes in personality and behavior, eventually rendering patients incapable of self-care.

Activation of microglia and astrocytes has been reported to be associated with the onset and progression of degenerative brain diseases. Microglia are the principal glial cells of the brain and spinal cord and play important roles in maintaining normal brain function and neuroprotection. Microglia contribute to the removal of excessive synapses as well as the clearance of amyloid beta (Aβ) and neurofibrillary tangles.

The role of microglia in the pathogenesis of Alzheimer's disease, which is the most common form of dementia, has been elucidated. For example, microglia or monocytes surrounding amyloid beta have been observed in the brains of Alzheimer's disease patients and mouse models. Microglia exert beneficial effects by removing amyloid beta through phagocytosis; however, they may also induce neuronal damage by causing excessive inflammatory responses.

Parkinson's disease is a common degenerative brain disease with a prevalence of approximately 1.2% in individuals aged 65 years or older. Neurodegeneration in Parkinson's disease occurs in the substantia nigra and is accompanied by dopaminergic denervation and accumulation of Lewy bodies containing alpha-synuclein. A large number of activated microglia have been identified in the parenchymal tissues of patients with Parkinson's disease, and microglia are known to mediate inflammatory responses in the central nervous system in Parkinson's disease.

Huntington's disease, which is caused by mutations in the huntingtin protein, is characterized by progressive atrophy of the striatum and cortex. In addition, pro-inflammatory microglia are observed at an early stage in this disease. The presence of pro-inflammatory microglia in Huntington's disease is closely associated with disease severity.

As the importance of the role of microglia in degenerative brain diseases has been elucidated, microglia have been proposed as a therapeutic target for the treatment of degenerative brain diseases.

Accordingly, the present inventors made efforts to develop a therapeutic agent for degenerative brain diseases that targets microglia while minimizing side effects associated with therapeutic administration and providing effective therapeutic efficacy. As a result, the inventors prepared a trimeric peptide composed of a very small peptide, which can minimize side effects associated with the administration of exogenous substances, and confirmed that the peptide induces microglia from a pro-inflammatory state to an anti-inflammatory state, thereby reducing inflammatory substances and increasing phagocytic activity to induce the removal of amyloid beta protein. In addition, in an animal model of a degenerative brain disease administered with the peptide, the inventors confirmed effects including reduction of amyloid beta, restoration of synaptic plasticity, and improvement of cognitive impairments associated with anxiety, learning, and memory. Accordingly, the present inventors completed the present application by demonstrating that the peptide of the present invention may be usefully employed as an active ingredient in a composition for the prevention or treatment of degenerative brain diseases and cognitive impairment, learning impairment, or memory impairment associated therewith.

### [Prior Art Documents]

### [Patent Documents]

Korean Patent Publication No. 10-2021-0055626

### [Non-Patent Documents]

Efthymiou, A.G. and A.M. Goate, Late onset Alzheimer's disease genetics implicates microglial pathways in disease risk. Mol Neurodegener, 2017. 12(1): p. 43.

Hong, S., et al., Complement and microglia mediate early synapse loss in Alzheimer mouse models. Science, 2016. 352(6286): p. 712-716.

Crotti, A. et al., Mutant huntingtin promotes autonomous microglia activation via myeloid lineage-determining factors. Nat. Neurosci. 17, 513-521(2014).

### [Disclosure of Invention]

### [Technical Problem]

An object of the present invention is to provide a composition for preventing, treating, or ameliorating a degenerative brain disease, comprising, as an active ingredient, a peptide consisting of the amino acid sequence represented by SEQ ID NO: 1, or a polynucleotide encoding the same.

Another object of the present invention is to provide a composition for preventing or treating cognitive impairment, learning impairment, or memory impairment, comprising, as an active ingredient, a peptide consisting of the amino acid sequence represented by SEQ ID NO: 1, or a polynucleotide encoding the same.

### [Solution to Problem]

In order to achieve the objects of the present invention, the present invention provides: a pharmaceutical composition for preventing or treating a degenerative brain disease, comprising, as an active ingredient, a peptide consisting of the amino acid sequence represented by SEQ ID NO: 1, or a polynucleotide encoding the same; use of the peptide or the polynucleotide encoding the same for use in a pharmaceutical composition for the prevention or treatment of a degenerative brain disease; use of the peptide or the polynucleotide encoding the same for the manufacture of a pharmaceutical composition for the prevention or treatment of a degenerative brain disease; and a method for treating a degenerative brain disease, comprising administering to a subject in need thereof the peptide or the polynucleotide encoding the same.

Further, the present invention provides: a health functional food composition for preventing or ameliorating a degenerative brain disease, comprising, as an active ingredient, a peptide consisting of the amino acid sequence represented by SEQ ID NO: 1, or a polynucleotide encoding the same; use of the peptide or the polynucleotide encoding the same for use in a health functional food composition for the prevention or amelioration of a degenerative brain disease; use of the peptide or the polynucleotide encoding the same for the manufacture of a health functional food composition for the prevention or amelioration of a degenerative brain disease; and a method for preventing or ameliorating a degenerative brain disease, comprising administering to a subject in need thereof the peptide or the polynucleotide encoding the same.

Further, the present invention provides: a pharmaceutical composition for preventing or treating cognitive impairment, learning impairment, or memory impairment, comprising, as an active ingredient, a peptide consisting of the amino acid sequence represented by SEQ ID NO: 1, or a polynucleotide encoding the same; use of the peptide or the polynucleotide encoding the same for use in a pharmaceutical composition for the prevention or treatment of cognitive impairment, learning impairment, or memory impairment; use of the peptide or the polynucleotide encoding the same for the manufacture of a pharmaceutical composition for the prevention or treatment of cognitive impairment, learning impairment, or memory impairment; and a method for preventing or treating cognitive impairment, learning impairment, or memory impairment, comprising administering to a subject in need thereof the peptide or the polynucleotide encoding the same.

Further, the present invention provides: a health functional food composition for enhancing cognitive ability, learning ability, or memory, comprising, as an active ingredient, a peptide consisting of the amino acid sequence represented by SEQ ID NO: 1, or a polynucleotide encoding the same; use of the peptide or the polynucleotide encoding the same for use in a health functional food composition for enhancing cognitive ability, learning ability, or memory; use of the peptide or the polynucleotide encoding the same for the manufacture of a health functional food composition for enhancing cognitive ability, learning ability, or memory; and a method for enhancing cognitive ability, learning ability, or memory, comprising administering to a subject in need thereof the peptide or the polynucleotide encoding the same.

### [Advantageous Effects of Invention]

In the present invention, it was confirmed that the trimeric peptide according to the present invention crosses the blood-brain barrier (BBB) and acts on microglia, which are the target cells. In addition, it was confirmed that the peptide induces microglia from a pro-inflammatory state to an anti-inflammatory state under disease conditions, thereby reducing neuroinflammation and increasing phagocytic activity to induce the removal of amyloid beta protein. Furthermore, reduction of amyloid beta was confirmed in an Alzheimer's disease animal model administered with the peptide. Therefore, the peptide of the present invention may be usefully employed as an active ingredient in a composition for preventing or treating degenerative brain diseases.

Further, in the present invention, restoration of synaptic plasticity and improvement of cognitive impairments associated with anxiety, learning, and memory were confirmed in an Alzheimer's disease animal model administered with the trimeric peptide according to the present invention. In addition, alleviation of memory-related cognitive impairment was confirmed in a dementia animal model administered with the peptide. Therefore, the peptide of the present invention may be usefully employed as an active ingredient in a composition for preventing or treating cognitive impairment, learning impairment, or memory impairment.

Furthermore, the trimeric peptide according to the present invention is composed of a very small peptide, thereby minimizing side effects associated with the administration of exogenous substances.

### [Brief Description of Drawings]

Fig. 1 is a schematic diagram illustrating a method of inducing an Alzheimer's disease environment in microglia and treating the microglia with the trimeric peptide KINE-101 prepared according to one embodiment of the present invention.
Fig. 2 shows the decreased expression of the pro-inflammatory state marker CD86 and the increased expression of the anti-inflammatory state marker CD206 after treatment of microglia cultured under an Alzheimer's disease environment with KINE-101 (ns, p>0.05; * p<0.05; ** p<0.01; *** p<0.001; **** p<0.0001, scale bar: 100 µm).
Fig. 3 shows the reduction in the production of the inflammatory mediator nitric oxide (NO) after treatment of microglia cultured under an Alzheimer's disease environment with KINE-101 (ns, p>0.05; * p<0.05; ** p<0.01; *** p<0.001; **** p<0.0001, scale bar: 100 µm).
Fig. 4 shows the intracellular transport of amyloid beta (Aβ) after treatment of microglia cultured under an Alzheimer's disease environment with KINE-101 (ns, p>0.05; * p<0.05; ** p<0.01; *** p<0.001; **** p<0.0001, scale bar: 100 µm).
Fig. 5 is a schematic diagram illustrating an experimental method for evaluating blood-brain barrier (BBB) permeability of KINE-101 in a mouse model.
Fig. 6 shows the BBB permeability of KINE-101 in a mouse model.
Fig. 7 shows the reduction of amyloid beta in the brain by KINE-101 in an amyloid beta-induced Alzheimer's disease mouse model (ns, p>0.05; *** p<0.001).
Fig. 8 is a schematic diagram illustrating administration of KINE-101 and behavioral testing in an amyloid beta-induced Alzheimer's disease mouse model.
Fig. 9 illustrates a method for performing an Elevated Plus Maze behavioral test using an amyloid beta-induced Alzheimer's disease mouse model.
Fig. 10 illustrates a method for performing a Novel Object Recognition Test (NORT) using an amyloid beta-induced Alzheimer's disease mouse model.
Fig. 11 illustrates a method for performing an Object Location Test (OLT) using an amyloid beta-induced Alzheimer's disease mouse model.
Fig. 12 shows the results of the Elevated Plus Maze behavioral test in an amyloid beta-induced Alzheimer's disease mouse model administered with KINE-101 (* p<0.05; ** p<0.01).
Fig. 13 shows the results of the Novel Object Recognition Test (NORT) in an amyloid beta-induced Alzheimer's disease mouse model administered with KINE-101 (* p<0.05).
Fig. 14 shows the results of the Object Location Test (OLT) in an amyloid beta-induced Alzheimer's disease mouse model administered with KINE-101 (* p<0.05).

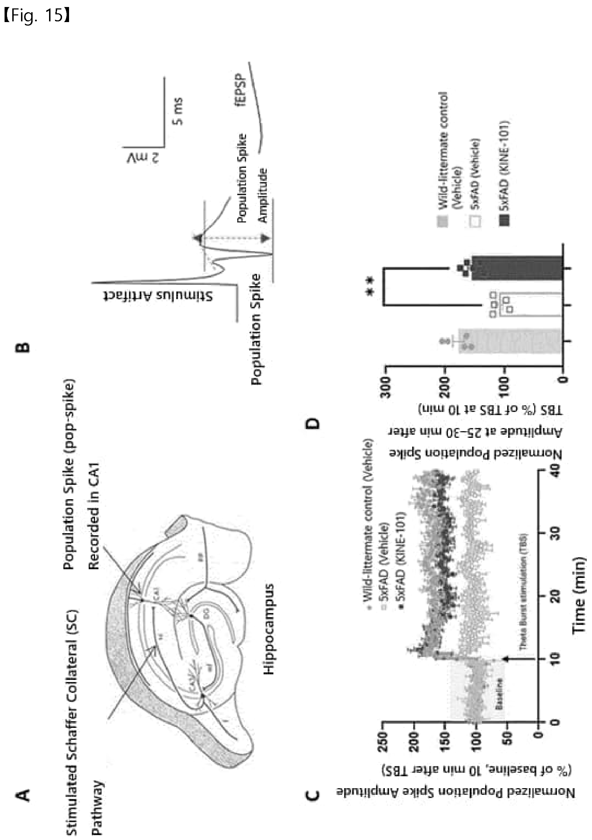

Fig. 15 shows a method for measuring long-term potentiation (LTP) following administration of KINE-101 to an amyloid beta-induced Alzheimer's disease mouse model, and the measurement results thereof. Specifically, Fig. 15A is a schematic diagram illustrating the hippocampus of a mouse and the mechanism of generating a population spike (pop-spike); Fig. 15B shows an example of a pop-spike and a method for measuring the amplitude thereof; Fig. 15C shows time-dependent changes in pop-spike amplitude according to administration of KINE-101 in an amyloid beta-induced Alzheimer's disease mouse model; and Fig. 15D shows recovery of LTP by KINE-101 in the amyloid beta-induced Alzheimer's disease mouse model (** p<0.01).
Fig. 16 shows a method for analyzing sharp-wave ripples (SWRs) following administration of KINE-101 to an amyloid beta-induced Alzheimer's disease mouse model, and the analysis results thereof. Specifically, Fig. 16A shows examples of SWRs according to frequency; Fig. 16B shows the increased occurrence frequency of SWRs induced by KINE-101 in an amyloid beta-induced Alzheimer's disease mouse model; and Fig. 16C shows the increased intensity of SWRs induced by KINE-101 in the amyloid beta-induced Alzheimer's disease mouse model (* p<0.05).
Fig. 17 shows a method for performing a Y-maze test using a scopolamine-induced dementia mouse model (Figs. 17A and 17B), and the results of the test (Fig. 17C) (ns, p>0.05; * p<0.05; **** p<0.0001; ## p<0.01).

### [Best Mode for Carrying Out the Invention]

Hereinafter, the present invention will be described in more detail.

The present invention provides a peptide consisting of the amino acid sequence represented by SEQ ID NO: 1, or a polynucleotide encoding the same.

In the present invention, the term "peptide" refers to a polymer composed of two or more amino acids linked by amide bonds (or peptide bonds). Despite various studies on peptide therapeutics, peptides themselves have exhibited disadvantages in that their excessively large size may induce antibody production and immune responses against the peptide drug itself, thereby reducing therapeutic efficacy and causing side effects. In this regard, the present invention is technically significant in that it identifies a trimeric peptide having pharmaceutically effective activity while consisting of 10 or fewer amino acids.

The peptide of the present invention may consist of the amino acid sequence represented by SEQ ID NO: 1, and may include an amino acid sequence having at least 75% sequence homology, preferably at least 80% sequence homology, more preferably at least 90% sequence homology, and most preferably at least 95% sequence homology with the amino acid sequence represented by SEQ ID NO: 1. More specifically, the peptide of the present invention may include an amino acid sequence having sequence homology of at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 99.5% with the amino acid sequence represented by SEQ ID NO: 1.

In addition, the peptide of the present invention may further comprise an amino acid sequence prepared for a specific purpose, such as a targeting sequence, a tag, a labeled residue, or for increasing the half-life or stability of the peptide.

In addition, the peptide of the present invention may be obtained by various methods widely known in the art. For example, the peptide may be prepared by recombinant polynucleotide techniques and protein expression systems, synthesized in vitro through chemical synthesis such as peptide synthesis, or prepared by cell-free protein synthesis methods.

In addition, in order to obtain improved chemical stability, enhanced pharmacological properties (e.g., half-life, absorbability, potency, efficacy, and the like), altered specificity (for example, a broad spectrum of biological activity), and reduced antigenicity, a protecting group may be conjugated to the N- or C-terminus of the peptide. Examples of the protecting group may include an acetyl group, a fluorenylmethoxycarbonyl group, a formyl group, a palmitoyl group, a myristyl group, a stearyl group, or polyethylene glycol (PEG). However, any component capable of modifying the peptide, particularly enhancing the stability of the peptide, may be included without limitation.

As used herein, the term "stability" refers not only to in vivo stability that protects the peptide of the present invention from attack by proteolytic enzymes in vivo, but also to storage stability (e.g., stability during storage at room temperature).

As used herein, the term "polynucleotide" refers to a polymer in which nucleotides are linked together and functions to transmit genetic information. For the purposes of the present invention, the polynucleotide encodes the peptide of SEQ ID NO: 1 and may include a sequence having at least 75% sequence homology, preferably at least 85% sequence homology, more preferably at least 90% sequence homology, and most preferably at least 95% sequence homology with the polynucleotide sequence encoding the peptide.

As used herein, the term "homology" refers to the degree of similarity with a wild-type amino acid sequence or polynucleotide sequence. Comparison of such homology may be performed using comparison programs widely known in the art, and the homology between two or more sequences may be calculated as a percentage (%).

Further, the present invention provides: a pharmaceutical composition for preventing or treating a degenerative brain disease, comprising, as an active ingredient, a peptide consisting of the amino acid sequence represented by SEQ ID NO: 1, or a polynucleotide encoding the same; use of the peptide or the polynucleotide encoding the same for use in a pharmaceutical composition for preventing or treating a degenerative brain disease; use of the peptide or the polynucleotide encoding the same for the manufacture of a pharmaceutical composition for preventing or treating a degenerative brain disease; and a method for treating a degenerative brain disease, comprising administering to a subject in need thereof the peptide or the polynucleotide encoding the same.

As used herein, the term "prevention" refers to any act of suppressing or delaying the onset of a disease by administration of the pharmaceutical composition according to the present invention.

As used herein, the term "treatment" refers to any act by which symptoms of a disease are ameliorated or beneficially altered by administration of the pharmaceutical composition according to the present invention.

As used herein, the term "subject" refers to an individual in need of treatment for a disease, and more specifically refers to mammals such as humans or non-human primates, rodents (e.g., mouse and rat), dogs, cats, horses, and cattle.

In the present invention, the degenerative brain disease may be dementia, Alzheimer's disease, Parkinson's disease, Huntington's disease, mild cognitive impairment, cerebral amyloid angiopathy, amyloid stroke, systemic amyloidosis, Dutch-type amyloidosis, Pick's disease, senile dementia, Lewy Body Dementia, Progressive Supranuclear Palsy, or Frontotemporal Dementia, but is not limited thereto.

In the present invention, the composition targets microglia and may prevent or treat the degenerative brain disease through the following properties by targeting microglia:
i) reducing neuroinflammation by inducing microglia from a pro-inflammatory state to an anti-inflammatory state; and
ii) removing amyloid beta through activation of phagocytosis by microglia.

Specifically, the composition may prevent or treat the degenerative brain disease by crossing the blood-brain barrier (BBB), targeting microglia in the brain, inducing inflammatory microglia to an anti-inflammatory state to reduce neuroinflammation, and increasing phagocytic activity to remove amyloid beta, which is a toxic protein.

In a specific embodiment of the present invention, the present inventors prepared KINE-101, a trimeric peptide, using a PSP fragment (PSP monomer), and confirmed that KINE-101 targets microglia and induces microglia from a pro-inflammatory state to an anti-inflammatory state, thereby reducing inflammatory substances and increasing phagocytic activity to induce removal of amyloid beta protein.

Further, the present inventors confirmed that KINE-101 reduces amyloid beta in the brain of an Alzheimer's disease mouse model, restores synaptic plasticity, and improves cognitive impairments associated with anxiety, learning, and memory. In addition, the inventors confirmed that KINE-101 alleviates memory-related cognitive impairment in a dementia mouse model.

Accordingly, the present inventors confirmed that the peptide of the present invention targets microglia, activates the phagocytic activity of microglia to remove amyloid beta, and induces inflammatory microglia to an anti-inflammatory state. In addition, the inventors confirmed that the peptide reduces amyloid beta, restores synaptic plasticity, and improves cognitive impairments associated with anxiety, learning, and memory in animal models of degenerative brain diseases. Therefore, the peptide of the present invention may be usefully employed as an active ingredient in a pharmaceutical composition for the prevention or treatment of degenerative brain diseases.

The peptide of the present invention or the polynucleotide encoding the same may be carried by a pharmaceutically acceptable carrier, such as a colloidal suspension, powder, saline, lipid, liposome, microsphere, or nanosphere. These may form complexes with or be associated with delivery vehicles, and may be delivered in vivo using delivery systems known in the art, such as lipids, liposomes, microparticles, gold, nanoparticles, polymers, condensation agents, polysaccharides, polyamino acids, dendrimers, saponins, absorption enhancers, or fatty acids.

In addition, the pharmaceutically acceptable carrier may include, but is not limited to, lactose, dextrose, sucrose, sorbitol, mannitol, starch, acacia gum, calcium phosphate, alginate, gelatin, calcium silicate, microcrystalline cellulose, polyvinyl pyrrolidone, cellulose, water, syrup, methyl cellulose, methyl hydroxybenzoate, propyl hydroxybenzoate, talc, magnesium stearate, mineral oil, and the like, which are commonly used in formulation preparation. Further, in addition to the above components, lubricants, wetting agents, sweeteners, flavoring agents, emulsifiers, suspending agents, preservatives, and the like may be additionally included.

The pharmaceutical composition of the present invention may be administered orally or parenterally (for example, by intramuscular, intravenous, intraperitoneal, subcutaneous, intradermal, or topical administration) according to the intended method. The dosage may vary depending on the condition and body weight of the patient, severity of the disease, dosage form, route of administration, and administration time, but may be appropriately selected by those skilled in the art.

The pharmaceutical composition of the present invention is administered in a pharmaceutically effective amount. As used herein, the term "pharmaceutically effective amount" refers to an amount sufficient to treat a disease at a reasonable benefit/risk ratio applicable to medical treatment. The effective dosage level may be determined depending on factors including the type and severity of the patient's disease, activity of the drug, sensitivity to the drug, administration time, route of administration, excretion rate, duration of treatment, concomitantly used drugs, and other factors well known in the medical field. The pharmaceutical composition according to the present invention may be administered alone or in combination with other therapeutic agents, and may be administered simultaneously, separately, or sequentially with conventional therapeutic agents. In addition, the composition may be administered in a single dose or multiple doses. Considering all of the above factors, it is important to administer an amount capable of achieving the maximum effect with the minimum amount without causing side effects, and such an amount may be readily determined by those skilled in the art.

Specifically, the effective amount of the pharmaceutical composition of the present invention may vary depending on the age, sex, condition, and body weight of the patient, absorption of the active ingredient in the body, inactivation rate, excretion rate, type of disease, and concomitantly administered drugs, and may be increased or decreased depending on the route of administration, severity of obesity, sex, body weight, age, and the like.

Further, the present invention provides: a health functional food composition for preventing or ameliorating a degenerative brain disease, comprising, as an active ingredient, a peptide consisting of the amino acid sequence represented by SEQ ID NO: 1, or a polynucleotide encoding the same; use of the peptide or the polynucleotide encoding the same for use in a health functional food composition for preventing or ameliorating a degenerative brain disease; use of the peptide or the polynucleotide encoding the same for the manufacture of a health functional food composition for preventing or ameliorating a degenerative brain disease; and a method for preventing or ameliorating a degenerative brain disease, comprising administering to a subject in need thereof the peptide or the polynucleotide encoding the same.

As used herein, the term "amelioration" refers to any act of at least reducing parameters associated with a condition to be treated, for example, the severity of symptoms.

In the present invention, since the descriptions of the peptide, polynucleotide, and degenerative brain disease are the same as those described above, detailed descriptions thereof will be omitted by reference to the foregoing descriptions, and only the specific components of the health functional food composition will be described below.

Meanwhile, the present inventors confirmed that the peptide of the present invention targets microglia, activates the phagocytic activity of microglia to remove amyloid beta, and induces inflammatory microglia to an anti-inflammatory state. In addition, the inventors confirmed that the peptide reduces amyloid beta, restores synaptic plasticity, and improves cognitive impairments associated with anxiety, learning, and memory in animal models of degenerative brain diseases. Therefore, the peptide of the present invention may be usefully employed as an active ingredient in a health functional food composition for the prevention or amelioration of degenerative brain diseases.

The health functional food composition of the present invention may be used for the prevention or amelioration of a disease before the onset stage of the disease or after disease onset, either simultaneously with or separately from a therapeutic agent for treatment.

In the health functional food composition of the present invention, the active ingredient may be added to a food as it is or may be used together with other foods or food ingredients, and may be appropriately used according to conventional methods. The amount of the active ingredient to be mixed may be suitably determined according to the intended use thereof (prevention or amelioration). In general, during the preparation of a food or beverage, the composition of the present invention may be added in an amount of preferably 15 wt% or less, more preferably 10 wt% or less, based on the raw materials. However, in the case of long-term intake for health and hygiene purposes or for health regulation purposes, the amount may be lower than the above range.

The health functional food composition of the present invention may further comprise other components as essential ingredients without particular limitation in addition to containing the active ingredient. For example, various flavoring agents or natural carbohydrates may be included as additional components, as in conventional beverages. Examples of the above-described natural carbohydrates include monosaccharides such as glucose and fructose; disaccharides such as maltose and sucrose; and polysaccharides such as conventional sugars including dextrin and cyclodextrin, as well as sugar alcohols such as xylitol, sorbitol, and erythritol. As flavoring agents other than those described above, natural flavoring agents (e.g., thaumatin and stevia extracts such as rebaudioside A and glycyrrhizin) and synthetic flavoring agents (e.g., saccharin and aspartame) may be advantageously used. The proportion of the natural carbohydrates may be appropriately determined by those skilled in the art.

In addition, the health functional food composition of the present invention may further comprise various nutrients, vitamins, minerals (electrolytes), flavoring agents such as synthetic flavoring agents and natural flavoring agents, coloring agents and filling agents (e.g., cheese and chocolate), pectic acid and the salts thereof, alginic acid and salts thereof, organic acids, protective colloidal thickeners, pH regulators, stabilizers, preservatives, glycerin, alcohol, carbonating agents used in carbonated beverages, and the like. These components may be used independently or in combination, and the proportion of such additives may also be appropriately selected by those skilled in the art.

Further, the present invention provides: a pharmaceutical composition for preventing or treating cognitive impairment, learning impairment, or memory impairment, comprising, as an active ingredient, a peptide consisting of the amino acid sequence represented by SEQ ID NO: 1, or a polynucleotide encoding the same; use of the peptide or the polynucleotide encoding the same for use in a pharmaceutical composition for preventing or treating cognitive impairment, learning impairment, or memory impairment; use of the peptide or the polynucleotide encoding the same for the manufacture of a pharmaceutical composition for the prevention or treatment of cognitive impairment, learning impairment, or memory impairment; and a method for preventing or treating cognitive impairment, learning impairment, or memory impairment, comprising administering to a subject in need thereof the peptide or the polynucleotide encoding the same.

As used herein, the term "cognitive impairment" refers to an abnormality in cognitive ability related to the manner of acquiring, remembering, and using knowledge. Cognitive ability includes cognitive functions such as knowledge, memory, comprehension, thinking ability, problem-solving ability, critical thinking ability, and creativity.

As used herein, the term "learning impairment" refers to a disorder characterized by significant difficulties in learning functions such as listening, speaking, attention, perception, memory, and problem-solving, or in academic achievement areas such as reading, writing, and mathematics.

As used herein, the term "memory impairment" refers to a condition in which newly acquired information cannot be remembered, the names of objects or people cannot be recalled, or recalling past experiences is difficult or impossible.

Such cognitive impairment, learning impairment, or memory impairment may be caused by various factors and environments, and the causes thereof are not particularly limited in the present invention. For example, they may result from degenerative brain diseases such as Alzheimer's disease, dementia, mild cognitive impairment, cognitive deficiency, and attention deficit.

In the present invention, since the descriptions of the peptide, polynucleotide, degenerative brain disease, and pharmaceutical composition are the same as those described above, detailed descriptions thereof will be omitted by reference to the foregoing descriptions.

Meanwhile, the present inventors confirmed that the peptide of the present invention restores synaptic plasticity and improves cognitive impairments associated with anxiety, learning, and memory in animal models of degenerative brain diseases. Therefore, the peptide of the present invention may be usefully employed as an active ingredient in a pharmaceutical composition for the prevention or treatment of cognitive impairment, learning impairment, or memory impairment.

Further, the present invention provides: a health functional food composition for enhancing cognitive ability, learning ability, or memory, comprising, as an active ingredient, a peptide consisting of the amino acid sequence represented by SEQ ID NO: 1, or a polynucleotide encoding the same; use of the peptide or the polynucleotide encoding the same for use in a health functional food composition for enhancing cognitive ability, learning ability, or memory; use of the peptide or the polynucleotide encoding the same for the manufacture of a health functional food composition for enhancing cognitive ability, learning ability, or memory; and a method for enhancing cognitive ability, learning ability, or memory, comprising administering to a subject in need thereof the peptide or the polynucleotide encoding the same.

In the present invention, since the descriptions of the peptide, polynucleotide, degenerative brain disease, and health functional food composition are the same as those described above, detailed descriptions thereof will be omitted by reference to the foregoing descriptions.

Meanwhile, the present inventors confirmed that the peptide of the present invention restores synaptic plasticity and improves cognitive impairments associated with anxiety, learning, and memory in animal models of degenerative brain diseases. Therefore, the peptide of the present invention may be usefully employed as an active ingredient in a health functional food composition for enhancing cognitive ability, learning ability, or memory.

Hereinafter, the present invention will be described in detail with reference to Examples.

However, the following Examples are provided only for illustrating the present invention, and the scope of the present invention is not limited thereto.

### <Example 1> Preparation of Peptide

In this Example, a trimeric polymer, KINE-101, was prepared using a PSP fragment (PSP monomer), as shown in [Table 1] below. Thereafter, the synthesized peptide was purified using high-performance liquid chromatography (SHIMADZU Prominence HPLC), and a Shiseido Capcell Pak C18 Column (4.6 × 50 mm) was used as the column. In addition, the molecular weight of the synthesized peptide was confirmed using a mass spectrometer (AXIMA Assurance, MALDI-TOF, Shimadzu).

**[Table 1]**

| Peptide Name | Amino Acid Sequence | Molecular Weight (MW) |
|---|---|---|
| KINE-101 | PSPPSPPSP(SEQ ID NO: 1) | 861.9 g/mol |

### <Example 2> Confirmation of Anti-inflammatory Activity and Enhanced Phagocytic Function of Microglia by KINE-101 in an Alzheimer's Disease Environment

Microglia are known to remove various foreign substances, including amyloid beta, through phagocytosis; however, they are also known to be involved in degenerative brain diseases through chronic inflammatory responses or excessive activation of synaptic pruning functions. Accordingly, the effects of KINE-101 were evaluated by targeting microglia cultured under an Alzheimer's disease environment.

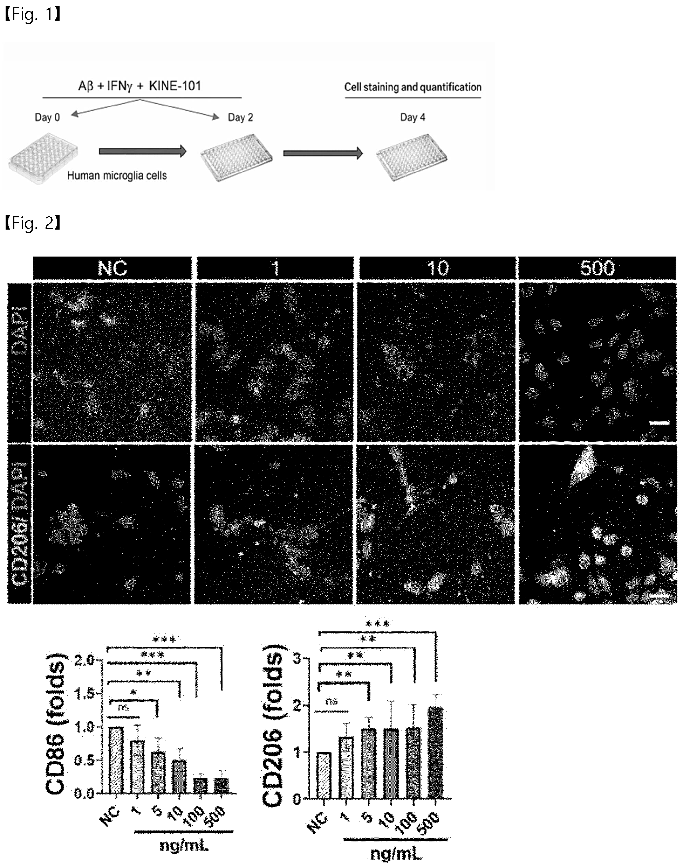

Specifically, as illustrated in the schematic diagram of Fig. 1, an Alzheimer's disease environment, which is the major cause of dementia, was established by treating cultured human microglia (hMG) with 1 µM oligomeric amyloid beta protein (Aβ), known to be a causative factor of Alzheimer's disease, and 10 ng/mL interferon-gamma (IFNγ), an inflammatory substance. Under these conditions, KINE-101 prepared in <Example 1> was treated at concentrations of 1, 5, 10, 100, or 500 ng/mL. After two days, the culture medium was replaced and the same treatment was repeated.

After an additional two days, in order to investigate whether the state of the microglia had shifted from a pro-inflammatory state (M1) to an anti-inflammatory state (M2), the cells were fixed using a fixative, and stained with antibodies against CD86, a marker of the pro-inflammatory state of microglia, and CD206, a marker of the anti-inflammatory state, followed by staining with fluorescence-labeled secondary antibodies. Images were obtained using a fluorescence microscope, and fluorescence signals were quantified using a software program to compare the degree of transition from the pro-inflammatory state to the anti-inflammatory state induced by KINE-101 (Fig. 2).

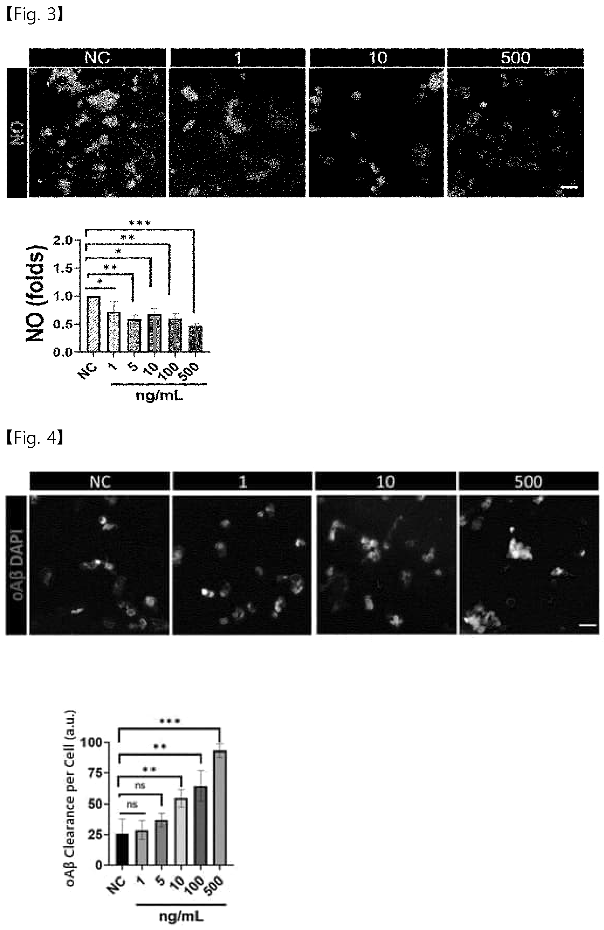

In addition, in order to evaluate the reduction of inflammatory substances by KINE-101, nitric oxide (NO), an inflammatory substance involved in the progression of Alzheimer's disease and present intracellularly, was stained with Difluorofluorescein-FM diacetate, and images were obtained using a fluorescence microscope. The intensity of the fluorescence signal was quantified and analyzed using a software program to confirm the degree of NO reduction induced by KINE-101 (Fig. 3).

Further, in order to investigate the degree of enhancement of microglial phagocytosis by KINE-101, intracellularly internalized amyloid beta protein was quantified. The amyloid beta protein was labeled with a pH-sensitive red fluorescent material, and thus exhibits fluorescence when transported to lysosomes, which function in protein degradation within cellular organelles, due to the low pH (pH 3-5) of the lysosomes. Therefore, the presence of intracellular red fluorescence indicates that amyloid beta has been internalized into microglia through phagocytosis and is being degraded in lysosomes. Red fluorescence images were obtained using a fluorescence microscope and quantified and compared using a software program (Fig. 4).

As a result, as shown in Fig. 2, KINE-101 concentration-dependently decreased CD86, a marker of the pro-inflammatory state of microglia cultured under an Alzheimer's disease environment, while increasing CD206, a marker of the anti-inflammatory state. These results indicate that KINE-101 has the ability to induce the state of microglia present in a pro-inflammatory state under a degenerative brain disease environment into an anti-inflammatory state.

Further, as shown in Fig. 3, KINE-101 concentration-dependently reduced NO, an inflammatory substance produced in microglia cultured under an Alzheimer's disease environment. These results indicate that KINE-101 exhibits anti-inflammatory efficacy by reducing inflammatory substances increased in microglia under a degenerative brain disease environment.

Further, as shown in Fig. 4, intracellular transport of amyloid beta in microglia cultured under an Alzheimer's disease environment increased in a concentration-dependent manner following treatment with KINE-101. These results indicate that KINE-101 exhibits efficacy in enhancing the phagocytic activity of microglia.

From the above results, it can be understood that KINE-101 targets microglia present in a pro-inflammatory state under an Alzheimer's disease environment and induces them to an anti-inflammatory state, thereby reducing inflammatory substances and enhancing phagocytic activity to induce the removal of amyloid beta protein.

### <Example 3> Confirmation of Blood-Brain Barrier (BBB) Permeability of KINE-101 in an Animal Model

In the treatment of degenerative brain diseases, the blood-brain barrier (BBB) acts as an obstacle that blocks the entry of therapeutic agents. Due to the structural specificity of cerebral capillaries, the BBB prevents most drugs from reaching target cells in the brain. Accordingly, in order to determine whether KINE-101 can cross the BBB and act on target cells in degenerative brain diseases, KINE-101 was administered to a mouse model and BBB permeability was evaluated.

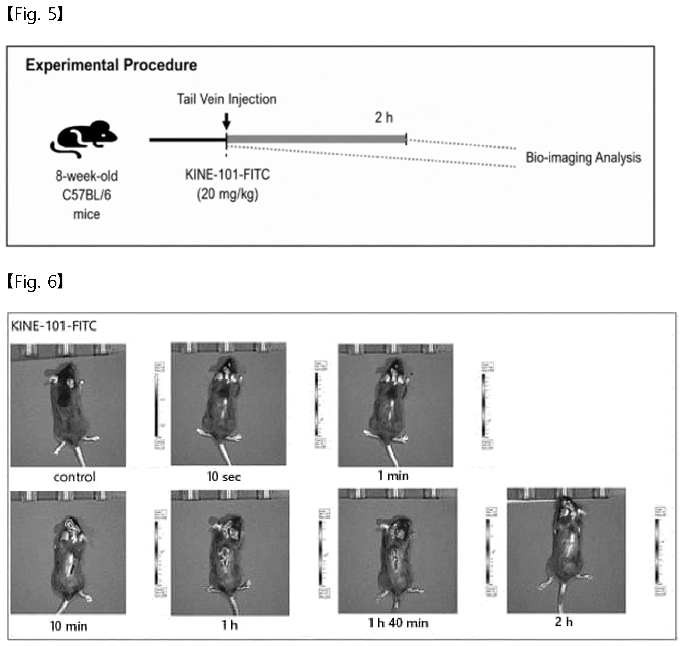

Specifically, as illustrated in the schematic diagram of Fig. 5, KINE-101 labeled with fluorescence (FITC) was intravenously administered at 20 mg/kg into the tail vein of 8-week-old male C57BL/6 mice, and fluorescence signals detected in the brain were monitored over time using a bio-imaging system (Fig. 6).

As a result, as shown in Fig. 6, fluorescence signals were observed in the brain region beginning 10 seconds after administration of KINE-101, and the strongest signal was observed after 10 minutes. In addition, the fluorescence signal was maintained for 2 hours.

From the above results, it was confirmed that KINE-101 is capable of crossing the BBB. Based on the *in vitro* results described above, it is expected that KINE-101 crossing the BBB may induce microglia present in the brain to an anti-inflammatory state, thereby reducing inflammatory substances and enhancing phagocytic activity to induce the removal of amyloid beta protein.

### <Example 4> Confirmation of the Effect of KINE-101 in an Animal Model of Degenerative Brain Disease

### <4-1> Confirmation of Reduction of Amyloid Beta Protein by KINE-101 in an Alzheimer's Disease Mouse Model

In order to evaluate the effect of KINE-101 in degenerative brain diseases, changes in amyloid beta expression were examined after administration of KINE-101 to an amyloid beta-induced Alzheimer's disease mouse model.

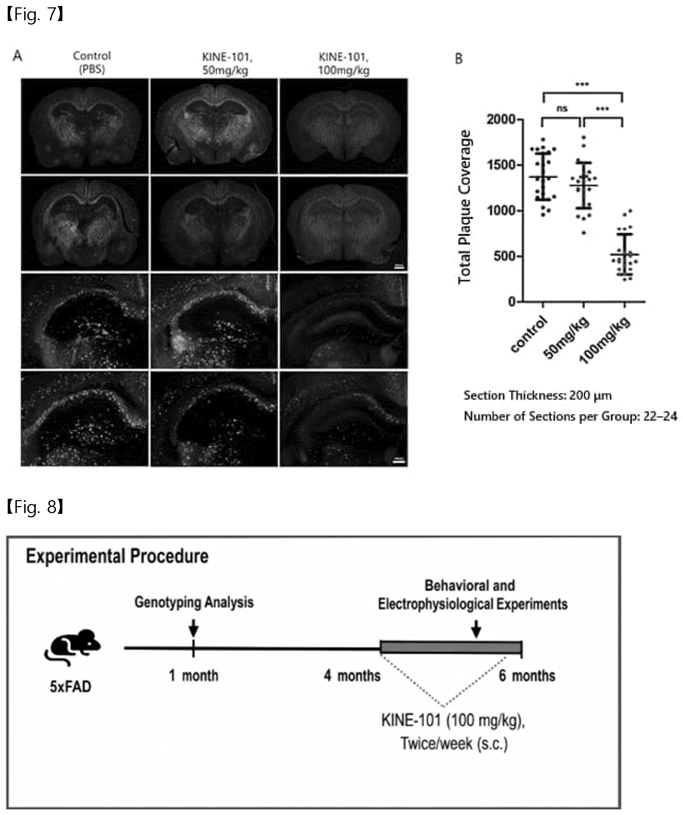

Specifically, the amyloid beta-induced Alzheimer's disease mouse model, 5xFAD mice, was obtained from Jackson Laboratory. This Alzheimer's disease mouse model is known to overexpress human amyloid beta protein, which undergoes extracellular oligomerization and is ultimately deposited as insoluble protein aggregates, thereby damaging neurons and causing dementia. Accordingly, KINE-101 synthesized in < Example 1> was administered intravenously through the tail vein of 4-month-old 5xFAD mice induced by amyloid beta at doses of 50 or 100 mg/kg, twice per week. After 4 weeks, brain tissues were sectioned into thin slices using a vibratome, and amyloid beta protein was probed using an antibody labeled with fluorescence (FITC dye). Images were obtained using a fluorescence microscope (Fig. 7A), and the amount of amyloid beta protein deposited in the brain tissues was quantified using Cytation5 software (Fig. 7B).

As a result, as shown in Fig. 7, a substantial amount of deposited amyloid beta protein was observed throughout the brain tissues of the 5xFAD mice. In contrast, amyloid beta protein was somewhat reduced in the brain tissues of mice administered with KINE-101 at 50 mg/kg. In the group administered with KINE-101 at a two-fold higher concentration of 100 mg/kg, it was confirmed that amyloid beta protein in the brain tissues was reduced by approximately 60% or more.

From the above results, it can be understood that KINE-101 is capable of crossing the BBB and removing toxic proteins deposited in the brain. In particular, considering the results showing increased uptake of amyloid beta protein by microglia induced by KINE-101, the reduction of amyloid beta protein in the mouse brain can be attributed to phagocytic activity of microglia activated by KINE-101.

### <4-2> Confirmation of Behavioral Improvement by KINE-101 in an Alzheimer's Disease Mouse Model

In order to evaluate the effect of KINE-101 on anxiety and impairments in learning and memory induced by degenerative brain diseases, behavioral changes were examined after administration of KINE-101 to an amyloid beta-induced Alzheimer's disease mouse model.

Specifically, as illustrated in the schematic diagram of Fig. 8, saline or KINE-101 synthesized in < Example 1> at a dose of 100 mg/kg was subcutaneously administered twice per week for a total of 2 months to 4-month-old 5xFAD mice induced by amyloid beta. In addition, normal littermate mice of the 5xFAD mice were used as a normal control group and were subcutaneously administered saline in the same manner.

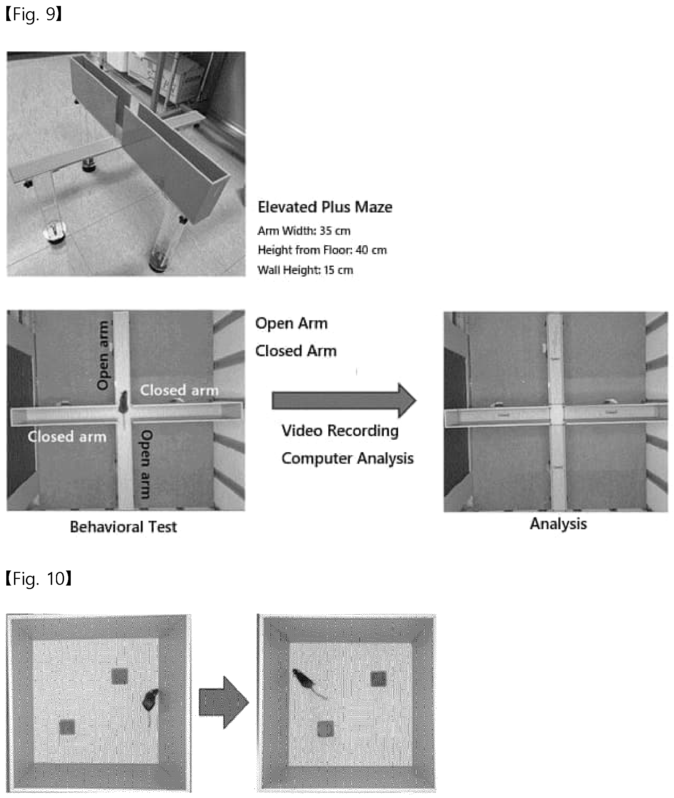

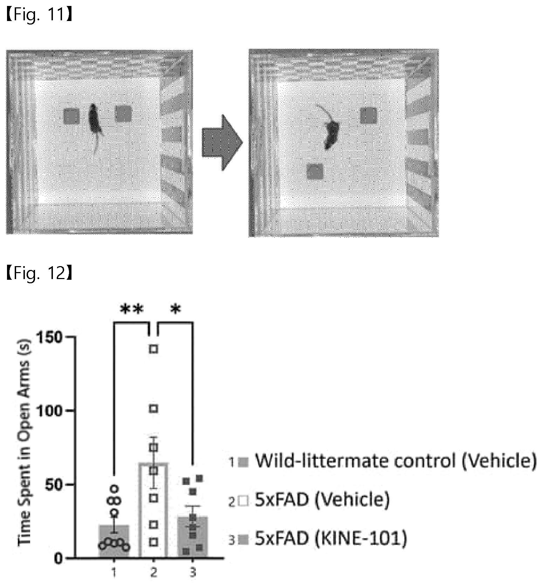

After completion of administration, an Elevated Plus Maze behavioral test was performed to evaluate anxiety-related behavior using an elevated cross-shaped maze, as shown in Fig. 9. The Elevated Plus Maze apparatus used in this experiment was designed to be positioned 40 cm above the floor surface, and among the four arms, two opposing arms were configured as closed arms with walls, while the remaining two arms were configured as open arms without walls. This behavioral test utilizes the innate tendency of chordatesto avoid elevated spaces without protective barriers due to the perceived threat of falling, and enables evaluation of anxiety-related behavior based on the duration of stay in the open arms, which induce a sense of threat, relative to the closed arms, which provide a greater sense of safety. Compared with the normal control group, extremely low preference for the open arms may be interpreted as a hyper-anxiety model, whereas high preference for the open arms may be interpreted as a hypo-anxiety disorder model. Alzheimer's disease animal models are known to exhibit hypo-anxiety behavior. Such behavior has been reported to be associated with interactions among the cerebrum, amygdala, and hippocampus involved in threat recognition and risk assessment. Prior to the behavioral test, experimental animals were subjected to a habituation process for at least 1 hour in the experimental environment, after which the animals were sequentially placed individually on the maze and allowed to freely explore without disturbance. The duration of stay in each arm was recorded by video for 5 minutes, and anxiety-related behavior was quantitatively compared by analyzing the time spent in the open arms without protective walls (Fig. 12).

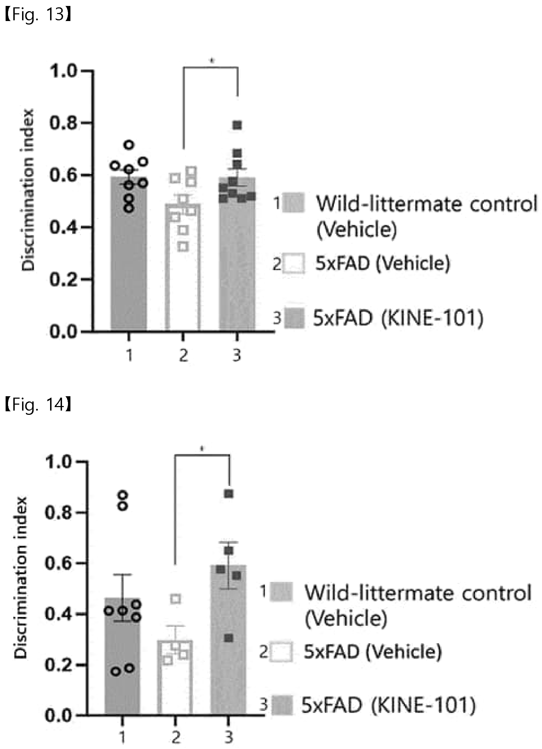

In addition, in order to investigate whether KINE-101 can improve memory-related cognitive impairment, a Novel Object Recognition Test (NORT) was performed to evaluate object recognition memory. NORT is a test method based on the tendency of experimental animals with innate exploratory behaviorto show greater interest in and spend more time investigating novel objects. As shown in Fig. 10, memory formation was induced by allowing the experimental animals to explore two objects placed in the test chamber. Thereafter, one of the objects was replaced with a novel object, and the time spent by the animals in remembering the previously encountered object and exploring the novel object was measured. In addition, a discrimination index was calculated according to the following equation (Fig. 13): Discrimination Index = time spent exploring the novel object / (time spent exploring the novel object + time spent exploring the familiar object).

Further, in order to determine whether spatial memory, which is a representative cognitive impairment observed in Alzheimer's disease patients, was improved, an Object Location Test (OLT) was performed. Similar to NORT, OLT allows experimental animals to explore two objects; however, instead of replacing one object with a novel object, only the location of one of the existing objects is changed, as shown in Fig. 11. This test method evaluates whether exploration time for the new location increases by inducing reorganization of place cells in the hippocampus as a result of object relocation in experimental animals with innate exploratory behavior. A discrimination index was calculated according to the following equation (Fig. 14): Discrimination Index = time spent exploring the relocated object location / (time spent exploring the relocated object location + time spent exploring the unchanged object location).

As a result, as shown in Fig. 12, evaluation of anxiety-related behavior demonstrated that the KINE-101-treated group [5xFAD(KINE-101)] exhibited a significantly shorter duration of stay in the open arms compared with the Alzheimer's disease-induced group [5xFAD(Vehicle)], and the duration was confirmed to be comparable to that of the normal control group [Veh-Littermate control (Vehicle)].

Further, as shown in Fig. 13, the results of the Novel Object Recognition Test (NORT) demonstrated that the KINE-101-treated group [5xFAD(KINE-101)] explored the novel object for a longer period of time compared with the Alzheimer's disease-induced group [5xFAD(Vehicle)] after administration for 2 months. These results indicate that administration of KINE-101 is effective in improving object recognition memory impairment.

Further, as shown in Fig. 14, the results of the Object Location Test (OLT) demonstrated that the KINE-101-treated group [5xFAD(KINE-101)] explored the new location to which the object had been moved for a longer period of time compared with the Alzheimer's disease-induced group [5xFAD(Vehicle)] after administration for 2 months. These results indicate that administration of KINE-101 is effective in improving spatial memory impairment.

From the above results, it can be understood that KINE-101 effectively improves anxiety and cognitive impairments associated with learning and memory observed in degenerative brain diseases such as Alzheimer's disease.

### <4-3> Confirmation of Increased Synaptic Plasticity and Associated Recovery of Cognitive Function by KINE-101 in an Alzheimer's Disease Mouse Model

In the formation of episodic memory, the transition from short-term memory to long-term memory necessarily requires the function of the hippocampus, a region of the brain, and is mediated by synaptic plasticity, which is a representative memory mechanism of the hippocampus. In particular, information transmission between neurons, the basic units of the brain, is mediated by synaptic plasticity, which is known to be a key mechanism underlying learning and memory formation. Accordingly, in order to evaluate the effect of KINE-101 on memory impairment induced by degenerative brain diseases, recovery of synaptic plasticity was examined following administration of KINE-101 to an amyloid beta-induced Alzheimer's disease mouse model.

Specifically, long-term potentiation (LTP) was measured by electrophysiological analysis using the mouse model of Example <4-2>. LTP was determined by comparing the amplitude of population-spikes (pop-spikes) before (baseline) and 25 minutes after application of theta burst stimulation (TBS), which is a short-term high-frequency stimulus. For LTP measurement, the mice were anesthetized, and electrical signals, namely population-spikes (pop-spikes), were induced in the hippocampus of the anesthetized mice (Fig. 15).

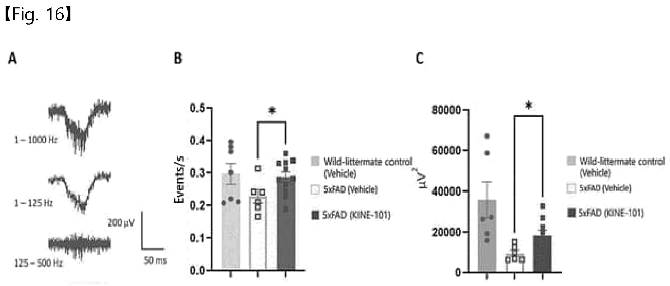

In addition, the replay mechanism, which is strengthened for the conversion of place cells into long-term memory, is observed in the form of sequential burst activation of hippocampal neurons when experimental animals cease exploration. Such phenomena are referred to as sharp waves (1-50 Hz) and ripples (125-250 Hz) according to the signal pattern, and collectively designated as sharp-wave ripples (SWRs). In order to determine whether administration of KINE-101 restores spatial memory formation ability in an Alzheimer's disease mouse model, the occurrence frequency and intensity of SWRs were comparatively analyzed. SWRs were determined by continuously measuring electrophysiological signals through electrodes implanted in the hippocampus, followed by measuring the occurrence frequency (events/s) and intensity (µV²) when signals within predetermined frequency ranges (sharp waves: 1-50 Hz; ripples: 125-250 Hz) exceeded the threshold value (4-fold the standard deviation) (Fig. 16).

As a result, as shown in Fig. 15, the LTP measurement results demonstrated that LTP was reduced in the Alzheimer's disease-induced group [5xFAD(Vehicle)] compared with the normal control group [Wild-Littermate control (Vehicle)], whereas the KINE-101-treated group [5xFAD(KINE-101)] exhibited increased LTP compared with the Alzheimer's disease-induced group. These results indicate that KINE-101 restores LTP and, consequently, restores synaptic plasticity.

Further, as shown in Fig. 16, SWR analysis demonstrated that the KINE-101-treated group [5xFAD(KINE-101)] exhibited increased frequency and intensity of SWRs compared with the Alzheimer's disease-induced group [5xFAD(Vehicle)]. These results suggest that KINE-101 contributes to recovery of SWRs, which reflect the activity of place cells involved in spatial memory.

From the above results, it can be understood that KINE-101 restores synaptic plasticity in the hippocampus and promotes the mechanism of place cell formation, thereby contributing to improvement of memory for objects and spatial information in degenerative brain diseases such as Alzheimer's disease.

### <4-4> Confirmation of the Effect of KINE-101 in a Dementia Mouse Model

In order to evaluate the effect of KINE-101 on learning and memory impairment caused by dementia as a degenerative brain disease, KINE-101 was administered to mice, followed by induction of dementia using scopolamine, and a Y-maze test was performed.

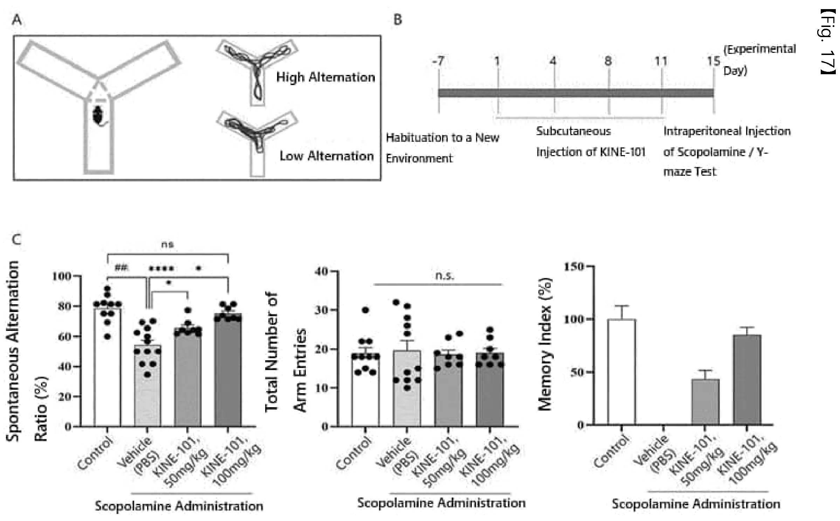

Specifically, scopolamine is a competitive antagonist that interferes with the binding of the neurotransmitter acetylcholine to acetylcholine receptors expressed in neurons, and is known to induce cognitive impairment, including learning and memory deficits, when administered to animals. In particular, scopolamine is known to activate microglia, thereby increasing inflammatory cytokines and oxidative stress and causing neuronal damage. In addition, the Y-maze test is a behavioral test designed based on the high exploratory tendency of mice toward novel environments and is used to evaluate working memory in animals (Fig. 17A). Accordingly, as illustrated in the schematic diagram of Fig. 17B, 8-week-old male C57BL/6 mice were divided into four groups, and KINE-101 (experimental group) or PBS (negative control group) was administered to each group by subcutaneous injection twice per week for 2 weeks under the conditions shown in Table 2. Four days later, scopolamine was administered by intraperitoneal injection. Thirty minutes after scopolamine administration, the mice were placed in a Y-shaped maze, and the sequence of movement through each arm during a 5-minute period was recorded. The percentage of spontaneous alternation (% Alternation) was quantified by calculating the number of entries into different arms (Fig. 17C).

**[Table 2]**

| Group | Administered Substance (Route of Administration) |
|---|---|
| Control | PBS(S.C.) |
| Vehicle(PBS) | PBS(S.C.) + Scopolamine (I.P., 1 mg/kg) |
| KINE-101, 50mg/kg | KINE-101 50 mg/kg(S.C.) + Scopolamine(I.P., 1 mg/kg) |
| KINE-101, 100mg/kg | KINE-101 100 mg/kg(S.C.) + Scopolamine(I.P., 1 mg/kg) |

As a result, as shown in Fig. 17, the scopolamine-treated mouse group [Vehicle (PBS)] exhibited a high frequency of movement into the same arm and thus a low spontaneous alternation ratio, whereas the positive control group [Control] exhibited a high frequency of movement into novel arms. In the KINE-101-treated mouse group [KINE-101], the spontaneous alternation ratio was statistically significantly increased, and a concentration-dependent tendency was observed. In particular, the mouse group administered with 100 mg/kg KINE-101 [KINE-101, 100 mg/kg] exhibited a spontaneous alternation ratio comparable to that of the positive control group. Meanwhile, the total number of arm entries was similar regardless of drug administration, indicating that the drug did not affect motor function in the mice and that the above results were not artifacts resulting from changes in motor activity. From these results, it can be understood that KINE-101 is capable of alleviating impairment of learning and memory induced by neuronal damage under inflammatory and oxidative stress conditions caused by scopolamine. In addition, KINE-101 may exert similar effects to alleviate cognitive impairment in other dementia-inducing degenerative brain diseases involving similar etiological environments.

### [Industrial Applicability]

The peptide of the present invention targets microglia and induces inflammatory microglia to an anti-inflammatory state under disease conditions, thereby reducing neuroinflammation and increasing phagocytic activity to promote removal of amyloid beta protein. In addition, the peptide exhibits effects including reduction of amyloid beta, restoration of synaptic plasticity, and improvement of cognitive impairments associated with anxiety, learning, and memory in animal models of degenerative brain diseases. Therefore, the peptide may be usefully employed as an active ingredient in a composition for the prevention or treatment of degenerative brain diseases and cognitive impairment, learning impairment, or memory impairment associated therewith.

## Claims

1. A pharmaceutical composition for preventing or treating a degenerative brain disease, comprising, as an active ingredient, a peptide consisting of the amino acid sequence represented by SEQ ID NO: 1, or a polynucleotide encoding the same.

2. The pharmaceutical composition according to claim 1, wherein an N- or C-terminus of the peptide is conjugated with a protecting group selected from the group consisting of an acetyl group, a fluorenylmethoxycarbonyl group, a formyl group, a palmitoyl group, a myristyl group, a stearyl group, and polyethylene glycol (PEG).

3. The pharmaceutical composition according to claim 1, wherein the degenerative brain disease is selected from the group consisting of dementia, Alzheimer's disease, Parkinson's disease, Huntington's disease, mild cognitive impairment, cerebral amyloid angiopathy, amyloid stroke, systemic amyloidosis, Dutch-type amyloidosis, Pick's disease, senile dementia, Lewy Body Dementia, Progressive Supranuclear Palsy, and Frontotemporal Dementia.

4. The pharmaceutical composition according to claim 1, wherein the composition is capable of crossing the blood-brain barrier (BBB).

5. The pharmaceutical composition according to claim 1, wherein the composition targets microglia.

6. The pharmaceutical composition according to claim 5, wherein the composition targets microglia and exhibits the following properties:
i) reducing neuroinflammation by inducing the microglia from a pro-inflammatory state to an anti-inflammatory state; and
ii) removing amyloid beta through activation of phagocytosis by the microglia.

7. The pharmaceutical composition according to claim 1, wherein the composition is formulated for oral administration, intramuscular administration, intravenous administration, intraperitoneal administration, subcutaneous administration, intradermal administration, or topical administration.

8. A health functional food composition for preventing or ameliorating a degenerative brain disease, comprising, as an active ingredient, a peptide consisting of the amino acid sequence represented by SEQ ID NO: 1, or a polynucleotide encoding the same.

9. A pharmaceutical composition for preventing or treating cognitive impairment, learning impairment, or memory impairment, comprising, as an active ingredient, a peptide consisting of the amino acid sequence represented by SEQ ID NO: 1, or a polynucleotide encoding the same.

10. A health functional food composition for enhancing cognitive ability, learning ability, or memory, comprising, as an active ingredient, a peptide consisting of the amino acid sequence represented by SEQ ID NO: 1, or a polynucleotide encoding the same.

11. A method for treating a degenerative brain disease, comprising administering to a subject in need thereof a peptide consisting of the amino acid sequence represented by SEQ ID NO: 1, or a polynucleotide encoding the same.

12. A method for preventing or ameliorating a degenerative brain disease, comprising administering to a subject in need thereof a peptide consisting of the amino acid sequence represented by SEQ ID NO: 1, or a polynucleotide encoding the same.

13. A method for preventing or treating cognitive impairment, learning impairment, or memory impairment, comprising administering to a subject in need thereof a peptide consisting of the amino acid sequence represented by SEQ ID NO: 1, or a polynucleotide encoding the same.

14. A method for enhancing cognitive ability, learning ability, or memory, comprising administering to a subject in need thereof a peptide consisting of the amino acid sequence represented by SEQ ID NO: 1, or a polynucleotide encoding the same.

15. Use of a peptide consisting of the amino acid sequence represented by SEQ ID NO: 1, or a polynucleotide encoding the same, for use in a pharmaceutical composition for the prevention or treatment of a degenerative brain disease.

16. Use of a peptide consisting of the amino acid sequence represented by SEQ ID NO: 1, or a polynucleotide encoding the same, for use in a health functional food composition for the prevention or amelioration of a degenerative brain disease.

17. Use of a peptide consisting of the amino acid sequence represented by SEQ ID NO: 1, or a polynucleotide encoding the same, for use in a pharmaceutical composition for the prevention or treatment of cognitive impairment, learning impairment, or memory impairment.

18. Use of a peptide consisting of the amino acid sequence represented by SEQ ID NO: 1, or a polynucleotide encoding the same, for use in a health functional food composition for enhancing cognitive ability, learning ability, or memory.

19. Use of a peptide consisting of the amino acid sequence represented by SEQ ID NO: 1, or a polynucleotide encoding the same, for the manufacture of a pharmaceutical composition for the prevention or treatment of a degenerative brain disease.

20. Use of a peptide consisting of the amino acid sequence represented by SEQ ID NO: 1, or a polynucleotide encoding the same, for the manufacture of a health functional food composition for the prevention or amelioration of a degenerative brain disease.

21. Use of a peptide consisting of the amino acid sequence represented by SEQ ID NO: 1, or a polynucleotide encoding the same, for the manufacture of a pharmaceutical composition for the prevention or treatment of cognitive impairment, learning impairment, or memory impairment.

22. Use of a peptide consisting of the amino acid sequence represented by SEQ ID NO: 1, or a polynucleotide encoding the same, for the manufacture of a health functional food composition for enhancing cognitive ability, learning ability, or memory.
